# EUROPEAN PATENT SPECIFICATION

(11) **EP 3 107 531 B1**
(45) Date of publication and mention of the grant of the patent: **03.01.2018**
(21) Application number: 15711861.3
(22) Date of filing: 20.02.2015
(51) Int. Cl.: A61K 9/20, A61K 9/68, A61K 31/728, A61K 47/36

(54) **NEW CHEWABLE COMPOSITION**
NEUE KAUBARE ZUSAMMENSETZUNG
NOUVELLE COMPOSITION A CROQUER

(30) Priority: 21.02.2014 IT RM20140079
(43) Date of publication of application: 28.12.2016
(73) Proprietor: IDI Integratori Dietetici Italiani S.r.l., 95020 Aci Bonaccorsi (CT) (IT)
(72) Inventor: BOTTINO, Pietro, I-95020 Aci Bonaccorsi (CT) (IT)
(74) Representative: Predazzi, Valentina
(86) International application number: PCT/IB2015/051311
(87) International publication number: WO 2015/125120

(56) References cited:
- WO-A1-2010/127250
- WO-A2-2010/136872
- M. D. JOHNSON ET AL: "Current therapies to shorten postoperative ileus", CLEVELAND CLINIC JOURNAL OF MEDICINE, vol. 76, no. 11, November 2009 (2009-11), pages 641-648, XP055131957, ISSN: 0891-1150, DOI: 10.3949/ccjm.76a.09051

## Description

The present invention relates to a new solid or semisolid composition to be chewed, comprising hyaluronic acid and/or salts thereof, for use in the treatment and/or prevention of postoperative paralytic ileus.

### PRIOR ART

Postoperative paralytic ileus (POI) is defined as the absence of peristalsis without mechanical obstruction, which occurs in the postoperative course of major abdominal surgery operations. It represents one of the main complications of iatrogenic etiology, onsetting in the postoperative period and manifests itself in a percentage ranging between 3 and 32% of patients undergoing abdominal or extra-abdominal surgical operation. Ramirez et al. (Ramirez JA, et al. "Definition, incidence, risk factors, and prevention of paralytic ileus following radical cystectomy: a systematic review." Eur. Urol. 2013 Oct;64(4):588-97) report a closer examination of POI-related issues, dwelling on clinical trials that make reference to paralytic ileus incidence. On 77 studies analyzed, 68 reported the incidence of POI. The rate of POI in cystectomy patients varied from 1.58% to 23.5%, with an average rate of 9.86% on a total of 13,793 patients.

In case of surgical operation, a physiological slowing down of intestinal functionality, of a duration varying from one to three days, is witnessed. A transient paralysis of bowel (intestinal) motility is considered, within certain time limits, physiological, the unavoidable consequence and response to surgical trauma undergone by viscera; specifically, the small intestine is the first one recovering normal motility within 24 hours; recovery of gastric motility normally has to occur within 24 - 48 hours, and last the colonic motor function, which takes 48 - 72 hours to be restored. If the alteration persists beyond 3 days, intestinal hypofunctionality is deemed pathological and falls within the clinical picture of postoperative ileus, which is all the more serious the longer the bowel dysfunction continues, on average 3 to 6 days after the operation.

The clinical picture of postoperative ileus is characterized by the reduction or complete absence of intestinal activities, in particular of gastrointestinal motility with entailed delay in gastric emptying and slowed-down or absent transit of gases and normoformed faeces in the intestine, and of the production of mucus and digestive secretions. Clinically, postoperative paralytic ileus manifests itself with nausea, vomiting, abdominal pain and distension, intolerance to orally intaken diet, patient discomfort often leading to the onsetting of a context of anxiety and prostration. Moreover, delayed canalization unavoidably entails a prolonging of patients' hospitalization, with an increase of health care costs and of the risk of development of nosocomial infections.

From the literature, it emerges that feasible interventions for preventing and reducing postoperative paralytic ileus are based on: reduction of the invasive character of the surgical operation, resorting to thoracic epidural analgesia, which reduces the need of opiates and acts on the sympathetic afferent pathways, controlled infusion of liquids, early enteral feeding within 24 hours following the operation.

Data in this regard are conflicting, as some literature data have highlighted that the resumption of early enteral feeding can cause vomiting and anastomosis rupture.

Chewing gum and postoperative ileus:
Recent literature data concur in upholding that gum chewing, starting from the first postoperative day, promotes a faster recovery of intestinal motility; whereas the results concerning chewing gum ability in reducing hospital stay days are conflicting.

It has been demonstrated, in an observational cohort study conducted on 102 patients subjected to cystectomy operation for a bladder-located tumour, as in patients who had received the chewing gum, compared to the control group, peristalsis resumption was faster (3.2 v 3.9 days; p < 0.001) and the time necessary for air (flatus) to pass again into the bowel (time to first flatus) was shorter (2.4 v 2.9 days; p < 0.0001), but days of hospital stay were not reduced (Kouba E. et al. "Gum Chewing Stimulates Bowel Motility in Patients Undergoing Radical Cystectomy with Urinary Diversion" Adult Urology 2007, pages 1053-1056)

These data were also confirmed by a systematic review and meta-analysis conducted by Edward et al. (Edward J. et al "Systematic review and meta-analysis of chewing-gum therapy in the reduction of post operative paralytic ileus following gastrointestinal surgery" World Journal of Surgery 2009, pages 2557-2566) in which seven studies were examined, for a total of 272 adult patients undergoing gastrointestinal surgery, and there were assessed: reduction of time to first passage of flatus in the bowel; reduction for first evacuation; reduction of length of hospital stay and of 30-day postoperative complications. Gum-chewing patients (144 pts), compared to those that had undergone routine postoperative treatment (128 pts.), exhibited reduction of time to first passage of flatus (12.6 hours) and to first evacuation (23.11 hours), but did not exhibit statistically significant differences in length of stay and appearance of postoperative complications.

The same aspects were evaluated in a prospective, double-blind randomized, placebo-controlled study, conducted on 66 patients undergoing colectomy (Matros E. et. al. "Does gum chewing ameliorate postoperative ileus? Results of a prospective, randomized, placebo-controlled trial" American College of Surgeons, 2006 202(5):773-8). Patients were subdivided into three groups and treated: the control group (21 pts) and the experimental group (22 pts) with chewing-gum, whereas the placebo group had to wear, at well-established times, a bracelet on the wrist. Time to first postoperative passage of flatus in the bowel occurred before in the experimental group (66 hours vs. 72/76 hours); as well as resumption of bowel peristalsis (86 hours vs. 96-87 hours). The experimental group, compared to the other two groups, did not show statistically significant differences for the other parameters considered, such as tolerance to two consecutive complete meals without complications, first postoperative evacuation and 30-day complications.

Further studies confirmed the decrease of time to first passage of flatus inside the bowel and the reduction in time of peristalsis resumption in gum-chewing patients, and moreover affirmed a decrease, though not statistically significant, in hospital stay.

Very recent works concur in affirming that chewing-gum represents a safe and economic method, apt to reduce the time to first passage of flatus and stimulate bowel motility in postoperative paralytic ileus.

M. D. Johnson et al: "Current therapies to shorten postoperative ileus",Cleveland Clinic Journal of Medicine, vol. 76, no. 11, November 2009, pages 641-648, discloses current therapies to shorten postoperative ileus, among which gum-chewing is pointed out.

Hyaluronic acid: hyaluronic acid is one of the main polysaccharide components of the extracellular matrix, abundantly present also in mesenchymal tissues of the embryo. Hyaluronic acid is one of the most hygroscopic molecules present in nature. The most important property of hyaluronic acid is therefore that of binding water, and this induces proteoglycans to become hydrated to the point of forming a gel-like structure.

Due to these properties, hyaluronic acid is widely used, as it forms an extracellular water film, maintains extracellular turgor, maintains tissue integrity, facilitates tissue regeneration and cell migration in case of inflammation.

Thanks to its hygroscopic, rheological and viscoelastic properties and to its biocompatibility, non-immunogenicity, biodegradability features, hyaluronic acid finds application in the cosmetic, medical and pharmaceutical fields.

Hyaluronic acid can be taken orally, injected or otherwise applied directly on the skin. Doses and application frequencies vary according to intended use. Intravenously injected hyaluronic acid is used to treat cases of knee or hip arthrosis, hyaluronic acid in the form of cream gel, spray, emulsion is also used to foster wound healing, to repair damages to skin and mucous membranes (such as burns, sores and ulcers). In these cases, the application has to be carried out directly on the concerned spot. Hyaluronic acid can also be taken orally for the treatment of various disorders of articular nature, and also finds application in plastic and cosmetic surgery.

The authors of the present invention have found that a chewable composition comprising hyaluronic acid and/or salts thereof can be effectively used in the prevention and/or treatment of postoperative paralytic ileus.

### SUMMARY OF THE INVENTION

The present invention therefore relates to a chewable solid or semisolid composition comprising hyaluronic acid and/or salts thereof, and to the use of the same for the prevention and/or treatment of postoperative paralytic ileus.

The prevention or treatment of postoperative paralytic ileus are currently addressed as follows:
a) Mechanical colon (bowel) preparation (MBP):
   This treatment envisages a preoperative mechanical colon preparation of the length of one or more days, associated with a low-waste diet to remove the fecal and bacterial content from the intestinal lumen. The rationale of MBP use is based on some theoretical advantages, among which enabling a quicker resumption of intestinal transit. Numerous clinical trials highlighted that MBP can be useless, in terms of reduction of infective complications and anastomotic dehiscences, and in some cases also detrimental, as entailing dehydration and electrolytic imbalances. Very recent evidence suggests that preoperative fasting may concur to determine paralytic ileus.
b) Positioning of nasogastric feeding tube:
   This treatment envisages its positioning during general anesthesia, and its removal once canalization has occurred. In the past, the nasogastric feeding tube was employed as standard practice in the postoperative period on patients undergoing abdominal surgery, to prevent the appearance of nausea and vomiting, avoid abdominal distension and foster early restoration of bowel functionality. Today it is used for decompressive purpose, in selected cases only and non-routinely. Its use has in fact been related to a higher incidence of hyperthermia, atelectasis, pulmonary complications, and to a delayed restoration of bowel functionality.
c) Fast-track approach or Enhanced Recovery After Surgery (ERAS):
   This is an alternative, multimode approach for the patient candidate to surgery, aimed at optimizing the perioperatory course with the objective of: abating surgical stress, obtaining a rapid recovery of the patient's abilities and limiting the frequency of main postoperative complications. At present, there is no standardized exclusive protocol, but rather a variety of treatment protocols are available, characterized by some points recognized as integral part of an advanced rehabilitation program (counseling interventions in the preoperative stage, absence of intestinal preparation, perioperative nutrition, early postoperative mobilization, etc.).
   Numerous studies and meta-analyses demonstrated the safety of the fast-track approach, and confirmed its advantages over traditional surgery, such as overall reduction of postoperative complications (tightness of anastomosis, intestinal occlusion, abscess formation) and postoperative ileus.
d) Gum-chewing:
   This approach promotes a faster recovery of bowel functionality, as chewing by itself stimulates the feeding processes; the action mechanism is the cholinergic vagal stimulation of the gastrointestinal tract.

Multiple literature evidence demonstrated that gum-chewing, despite anticipating the so-called first intestinal movement (propulsive activity of the loops), the canalization of gases (flatus) in the intestine and the first defecation, has no clinical relevance in the matter of the improvement of postoperative paralytic ileus and is not capable of reducing hospital stay times.

From what is reported above, it is evident that the solutions currently in use are susceptible of vast improvements.

The solution proposed in the present invention is based on a chewable composition comprising hyaluronic acid and/or salts thereof for oral use in the treatment and/or prevention of postoperative paralytic ileus. Said composition, besides performing the effects reported in the literature, related to chewing-gum use to restore intestinal (bowel) movement and canalization in the postoperative paralytic ileus, fosters, by the action performed by the hyaluronic acid, the resumption of peristalsis in patients that have undergone a surgical operation and enables the prevention or treatment of paralytic ileus, and consequently the reduction of postoperative complications.

The technical advancement proposed by the invention with respect to the prior art resides in the use of hyaluronic acid and/or salts thereof, associated to the use of a chewable solid or semisolid composition, for the treatment of paralytic ileus. The authors of the present invention have found that the chewing of a composition comprising hyaluronic acid and/or salts thereof suitable for alimentary or pharmaceutical use, by hydrating the buccal mucous membrane, not only reduces post-anesthesia mouth dryness, fostering chewing in operated subjects, but stimulates salivation, fostering gastric secretion and consequently peristalsis.

Preliminary data obtained on a group of 36 informed patients, in which one half of the patients used the composition of the invention in the form of chewing gum four times per day for 15 minutes after the abdominal operation and the other half used a gum not comprising hyaluronic acid or salts thereof, showed that the patients treated with the composition of the invention noticed a good salivation following gum use (whereas the others noticed mouth dryness). The same preliminary data show that the use of the composition of the invention seems to lead to a reduction of the times of resumption of the intestinal function and hospital stay times.

Moreover, the composition proved absolutely tolerable and pleasant to use for the patients.

The composition of the invention entails the advantages of being a safe and effective tool, without adverse effects, for the treatment of postoperative paralytic ileus.

Thanks to the hydrating action of hyaluronic acid, it concomitantly allows to reduce post-anesthesia mouth dryness, improve the act of chewing (made otherwise difficult) and foster intestinal peristalsis

The treatment is not invasive and not painful, has a high compliance for the patient and enables a marked saving for the National Health Service.

### DETAILED DESCRIPTION OF THE INVENTION

The present invention relates to a chewable solid or semisolid composition comprising hyaluronic acid and/or salts thereof for use in the treatment and/or prevention of postoperative paralytic ileus.

The chewable composition could be, e.g., in the form of chewable tablets, oromucosal capsules to be chewed, chewing gums, candies or chewing gums with a liquid core, gummy candy, chewable tablet.

When the composition is in the form of chewing gum, this may be any one type of chewing gum composition known to the technician in the field, and could be in the form of bar, strip, lozenge, tablet, ball, pill, filled pill, and could be coated or not coated.

The chewable composition according to the invention could be made according to any technique known in the field and with conventional components not requiring specific teachings for the technician in the field.

To prepare the composition, in any of the above-indicated forms, hyaluronic acid and/or any one hyaluronic acid salt suitable for oral use could be used, like, e.g., the sodium salt of hyaluronic acid (sodium hyaluronate). It could be used in solid form (powder, granules) or in liquid form (a form that can be particularly suitable in compositions comprising a core in the form of liquid or gel).

The hyaluronic acid or salt thereof suitable to be used for preparing the chewable composition of the invention could be hyaluronic acid or a salt thereof having a high, medium or low molecular weight; whose molecular weights are respectively within the following ranges: 1.800-2.200 KDa, 1000-1800 kDa, 100-500 KDa.
The hyaluronic acid and/or salt thereof will be present in the composition of the invention at a concentration between 0.01% and 0.7% by weight of the composition, like, e.g., between about 0.05% and 0.5% by weight, or between about 0.1 % and 0.2% by weight of the composition.

According to one embodiment of the invention, the hyaluronic acid and/or salts thereof will be present at a percentage by weight between 0.05 and 0.5% of the composition, e.g. 0.05%, 0.06%, 0.07%, 0.08%, 0.09%, 0.1%, 0.11%, 0.12%, 0.13%, 0.14%, 0.15%, 0.16%, 0.17%, 0.18%, 0.19%, 0.2%, 0.21%, 0.22%, 0.23%, 0.24%, 0.25%, 0.26%, 0.27%, 0.28%, 0.29%, 0.3%, 0.31 %, 0.32%, 0.33% 0.34%, 0.35%, 0.36%, 0.37%, 0.38%, 0.39%, 0.4%, 0.41 % 0.42%, 0.43%, 0.44%, 0.45%, 0.46%, 0.47%, 0.48%, 0.49%, 0.5%. In one embodiment of the invention, the hyaluronic acid and/or salt thereof may be at a concentration between 0.05 and 0.2%, like, e.g., 0.05, 0.06%, 0.07%, 0.08%, 0.09%, 0.1 %, 0.11 %, 0.12%, 0.13%, 0.14%, 0.15%, 0.16%, 0.17%, 0.18%, 0.19%, 0.2%.

In particular, the composition according to the present invention may comprise from about 0.17 mg to about 12 mg of hyaluronic acid and/or salts thereof per unit dose (by "unit dose" it is meant the single chewing gum, tablet, candy, oromucosal capsule), like e.g. about 0.5 mg, 1 mg, 1.5 mg, 2 mg, 2.5 mg, 3 mg, 3.5 mg, 4 mg, 4.5 mg, 5 mg, etc., per unit dose.

In a preferred embodiment, the composition comprises between 2.5 and 5.5 mg of hyaluronic acid and/or salts thereof per unit dose, like, e.g., about 2.5 mg, about 3 mg, about 3.5 mg, about 4 mg, about 4.5 mg, about 5 mg, about 5.5 mg per unit dose.

In case the composition is a chewable tablet, this will moreover comprise excipients normally usable by the expert in the field for the preparation of chewable tablets, like, e.g., thickeners, sweeteners, anti-caking agents, colouring agents, coating agents, aromatizing agents.

For instance, tablets, oromucosal capsules or gummy candies may contain conventional excipients, including, e.g., binding agents, like gum arabic, gelatine, sorbitol, gum tragacanth, and/or polyvinylpyrrolidone; fillers, like lactose, sugar, corn starch, rice starch, calcium phosphate, sorbitol and/or glycine; tableting lubricants, like magnesium stearate, talc, polyethylenglycol and/or silica; disintegrants, e.g. potato starch; and wetting agents like sodium laurylsulphate. The tablets can be coated according to methods well-known in standard pharmaceutical practice.

According to one embodiment, the composition could be made in the form of chewing gum. The composition as chewing gum can be made according to conventional methods known to the technician in the field.

For instance, the composition may comprise a percentage of between 25 and 35% of gum base, which is the non-nutritive water-insoluble inert support to which the edible and soluble components of chewing gums bind. Such a support normally comprises elastomers, providing elasticity to the gum, and they can be natural latexes such as *couma macrocarpa* (also called caspi or sorva), loquat (also called nispero), tunu, jelutong, or chicle, or synthetic rubbers such as styrenebutadiene rubber, butyl rubber, polyisobutylene and other commonly used ones. Moreover, resins are commonly present, providing a cohesive force or body of the gum, which are most often glycerol esters of gum, resins and/or polyvinyl acetate. Lastly, also waxes are present, which act as softening agents, like e.g. paraffin or microcrystalline wax.

The composition in the form of chewing gum moreover normally contains one or more of anti-caking agents, sweeteners, thickeners, aromas, any colouring agents and coating agents; for the carrying out of the invention, components conventionally used for the preparation of chewing gums are suitable.

By way of example, the anti-caking agents may be magnesium stearate, talc, polyethylenglycol and/or silica or mixtures thereof, the thickeners may be gum arabic, gum tragacanth or mixtures thereof; the sweeteners may be isomalt, xylitol, mannitol, sorbitol, sucralose, acesulfame, aspartame, stevia, or mixtures thereof.

In a particular embodiment, the composition could be made with biodegradable components, like e.g. rubber from Sapodilla tree (*Manilkara zapota, Manilkara* chicle, *Manikara bidentata*) or also containing cereal-derived proteins as described in patent application US5672367.

In one embodiment of the invention, the chewable composition according to the invention is a composition without lactose, gluten and/or aspartame.

According to a particular embodiment of the invention, the composition will comprise at least one dry extract of *Plantago psyllium.*

In terms of weight, the single dose could comprise from about 34 mg to about 170 mg of extract of *Plantago psyllium* per unit dose (i.e., single chewing gum, tablet, candy, oromucosal capsule), e.g. 70, 80, 90, 100, 110, 120 mg per unit dose, or even, e.g., 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110 mg of extract per unit dose.

In one embodiment of the invention the composition is a chewing gum comprising hyaluronic acid and/or salts thereof in the above-defined percentages.

In one embodiment, the chewing gum will have a weight of between 15 and 20 grams, hyaluronic acid and/or salts present in each gum could be, e.g., between 1.5 and 3.5 mg of hyaluronic acid and/or salts thereof for gum (unit dose), like, e.g., 1.5 mg, about 2 mg, about 2.5 mg, about 3 mg, about 3.5 mg per gum.

By way of example, the composition of the invention can also be made as chewable tablet, and can comprise, besides the ingredients typically used for the preparation of chewable tablets (thickeners, anti-caking agents, sweeteners, aromas), a percentage of hyaluronic acid and/or salts thereof between 0.4 and 0.7%.

In the chewable tablet, in fact, since the duration of chewing is usually less than that had for a gum, the concentration of hyaluronic acid and/or salts thereof can be higher than that used in the chewing gum.

According to a further embodiment, the above-described gum or chewable tablet could comprise a dry extract of *Plantago psyllium* between 2 and 10% by weight of the composition, e.g. between 80 and 110 mg per gum, e.g. 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110 mg of extract per gum. The chewable composition according to the invention, as mentioned above, comprises hyaluronic acid and/or salts thereof, and could be administered for the treatment or prevention of postoperative paralytic ileus in one or more unit doses throughout the day, e.g. 1, 2, 3, 4, or 5 unit doses (i.e., 1, 2, 3, 4, or 5 chewing gums, tablets, candies or oromucosal capsules), with a suggested chewing time of between 2 and 20 minutes.

According to one embodiment the composition could be used in the form of chewing gum with a posology of one chewing gum 3-4 times per day and a chewing time of between 5 and 20 minutes per gum. It is also disclosed a therapeutic method for the prevention or treatment of postoperative paralytic ileus which provides the administration of the chewable composition according to the invention from 1 to 5 times per day, e.g. between 2 and 4 times per day, e.g. 3 or 4 times per day, with a chewing time of between 2 and 20 minutes per administration.

The composition of the invention in any of the embodiments described herein could be a pharmaceutical, herbal, homeopathic composition, could be in the form of medical device, food for special medical purposes (medical food), of food supplement.

Hereinafter, examples of compositions according to the invention are provided which however are not binding, but have the aim of exemplifying some best embodiments of the invention; examples for the preparation of the composition and preliminary data on the effectiveness thereof are also provided.

Let us remind the fact that the preparation techniques of the chewable compositions described herein are standard techniques well-known to the technician in the field and need no exemplification in order to make the compositions of the invention.

### EXAMPLES

### 1. Formulation examples of the composition of the invention:

### 1.1 Composition example 1 CHEWING GUM

Gum base 25-35%
Sweeteners 53-63%
Thickeners 1-2%
Aromas 1-3%
Anti-caking agent 2.5-4.5%
Hyaluronic acid or a salt thereof 0.1-0.2%
Colouring agents 0-1%
Coating agents 0-0.5%

The components may be one or more of the components listed in the above description.
Hyaluronic acid may be present as salt sodium hyaluronate.

### 1.2 Composition example 2 CHEWING GUM

Gum base 25-35%
Sweeteners 53-63%
Thickeners 1-2%
Aromas 1-3%
Anti-caking agents 2.5-4.5%
Hyaluronic acid or salt thereof 0.1-0.2%
Colouring agents 0-1%
Coating agents 0-0.5%

The compositions may be one or more of the components listed in the above description.
Hyaluronic acid may be present as salt sodium hyaluronate.

### 1.3 Composition example 3 CHEWING GUM

Gum base 25-35%
Sweeteners 53-63%
Thickeners 1-2%
Aromas 1-3%
Anti-caking agents 2.5-4.5%
Plant extracts 4.5-6.5%
Hyaluronic acid or a salt thereof 0.1-0.2%
Colouring agents 0-1%
Coating agents 0-0.5%

The compositions may be one or more of the components listed in the above description.
Hyaluronic acid may be present as salt sodium hyaluronate.

Composition example 4 CHEWABLE TABLET
Thickeners 50-70%
Sweeteners 25-35%
Anti-caking agent 4-9%
Hyaluronic acid or a salt thereof 0.4-0.7%
Aromas 2-3%
Colouring agents 0-0.5%

Composition example 5 CHEWABLE TABLET
Thickeners 55-70%
Sweeteners 25-35%
Anti-caking agent 4-9%
Hyaluronic acid or a salt thereof 0.4-0.7%
Plant extracts 4.5-6.5%
Aromas 2-3%
Colouring agents 0-0.5%

According to the examples provided above, the chewing gum or chewable tablet compositions can comprise or consist in the above-listed ingredients.

### 2. Example of preparation of the composition as chewing gum

The raw materials for the gum base are processed so as to prepare the gum base according to standard processes, then there are added the other ingredients, such as sweeteners, aromas, thickeners, colouring agents, hyaluronic acid and/or salts thereof, optional plant extracts; the compound is prepared in the form of powder. The anti-caking agents are added, proceeding to materials compression and optional coating.
In case of liquid- or gel core gums, hyaluronic acid can be introduced into said core.

### 3. Experimental example of protocol used to assay the effectiveness of the composition of the invention.

### PRELIMINARY DATA - STUDY WITH CHEWING GUM

### 1. STUDY SAMPLE

36 patients
(18 patients treated with chewing gum according to example 1, 18 patients treated with standard chewing gum).

### 2. Data collected in two different centers. Sample randomization

### 3. INCLUSION CRITERIA

- Patients candidate to radical cystectomy surgery
- Age > 18 years
- Ability to carry out study requests
- Ability to provide informed consent in accordance with ICH/GCP good clinical practice rules, and national regulations in force

### 4. EXCLUSION CRITERIA

- Age < 18 years
- Anesthesiological risk class ASA IV
- Operations performed under urgency conditions
- Patients unable to understand what is proposed and to observe postoperative prescriptions
- Patients without a capable and responsible home care assistant
- Patients deemed unable to guarantee house hygienic conditions compatible with postoperative prescriptions
- Patients with ascertained intolerance to one or more components present in chewing gums according to example 1, or in over-the-counter ones.

### 5. TREATMENT POSOLOGY AND DURATION:

### Posology:

ARM I: chewing gum according to the invention, 1 for 15 minutes every 3 hours (for a total of 4 per day)
ARM II: commercial chewing gum, 1 for 15 minutes every 3 hours (for a total of 4 per day)

Treatment duration: from the first day post-intervention to the third day post-intervention.

### 6. ASSESSMENT CRITERIA ADOPTED:

- Average time to hospital discharge
- Average time to refeeding
- Palatableness and degree of patient's compliance
- chewing gum tolerability

### 7. RESULTS

- All patients who had received the chewing gums according to the invention took the 12 chewable gums.
- In the group that took the commercial chewing gums:
   - four patients interrupted chewing on the first postoperative day due to the onset of nausea
   - two patients exhibited signs of occlusion on the third postoperative day. Reintervention necessary (mechanical ileus from loop debridement into neobladder according to Bricker)
- Average time to hospital discharge: preliminary data indicate a trend toward faster hospital discharge in the group of patients treated with the gums according to the invention.
- Average time to refeeding: 3.5 days. In the group treated with the gums according to the invention: 2.7 days. The level of liking and palatableness was equal to 100% in the group treated with the gums according to the invention; 60% was reached in the control group.
- 90% of patients who took the gums according to the invention declared salivation increase and a feeling of hydration of the oral mucous membrane; in the control group, 70% of patients declared mouth dryness following a chewing carried on beyond 10 minutes.
- Good tolerability in both groups. No adverse reaction was observed.
The preliminary data herein, which were obtained as stated above on patients who gave their informed consent to the experimentation, show the effectiveness of the composition of the invention; it is in fact demonstrated how the gum according to the invention has better effects on intestinal refeeding time compared to commercial gums, and preliminary data indicate that average times of hospital discharge seem to be shorter in patients treated with the gums according to the invention.

## Claims

1. A chewable solid or semisolid composition comprising hyaluronic acid and/or a salt thereof for use in the treatment and/or prevention of postoperative paralytic ileus.

2. The chewable composition for use in the treatment and/or prevention of postoperative paralytic ileus according to claim 1 wherein said hyaluronic acid and/or a salt thereof is at a concentration between 0.01% and 0.7% by weight of said composition.

3. The chewable composition for use in the treatment and/or prevention of postoperative paralytic ileus according to claim 2 wherein said hyaluronic acid and/or a salt thereof is at a concentration between 0.05% and 0.5% by weight of said composition, or between 0.1 % and 0.3% by weight of said composition.

4. The chewable composition for use in the treatment and/or prevention of postoperative paralytic ileus according to any one of claims 1 to 3 wherein said salt of hyaluronic acid is sodium hyaluronate.

5. The chewable composition for use in the treatment and/or prevention of postoperative paralytic ileus according to any one of claims 1 to 4 wherein the amount of said hyaluronic acid and/or salt thereof is between 0.17 mg and 12 mg per unit dose.

6. The chewable composition for use in the treatment and/or prevention of postoperative paralytic ileus according to claim 5 wherein the amount of said hyaluronic acid and/or salt thereof is between 0.5 and 5.5 mg per unit dose.

7. The chewable composition for use in the treatment and/or prevention of postoperative paralytic ileus according to any one of claims 1 to 6 further comprising a dry extract of *Plantago Psyllium* at a concentration of between 2 and 10% by weight of said composition.

8. The chewable composition for use in the treatment and/or prevention of postoperative paralytic ileus according to claim 7 wherein said dry extract is at a concentration of about 4%, 5%, 5.5%, 6%, 6.5% by weight of said composition.

9. The chewable composition for use in the treatment and/or prevention of postoperative paralytic ileus according to claim 7 or 8 wherein the amount of said dry extract is between 65 and 111 mg per unit dose.

10. The chewable composition for use in the treatment and/or prevention of postoperative paralytic ileus according to any one of claims 1 to 9 in the form of chewing gum, chewable tablet, candy or oromucosal capsule.

11. The chewable composition for use in the treatment and/or prevention of postoperative paralytic ileus according to claim 10 wherein said composition is a chewing gum comprising as percentage by weight of said composition:
Gum base 25-35%
Sweeteners 53-63%
Thickeners 1-2%
Aromas 1-3%
Anti-caking agents 2.5-4.5%
Hyaluronic acid and/or a salt thereof 0.1-0.2%
Colouring agents 0-1%
Coating agents 0-0.5%.

12. The chewable composition for use in the treatment and/or prevention of postoperative paralytic ileus according to claim 10 wherein said composition is a chewing gum comprising as percentage by weight of said composition:
Gum base 25-35%
Sweeteners 53-63%
Thickeners 1-2%
Aromas 1-3%
Anti-caking agents 2.5-4.5%
Hyaluronic acid and/or a salt thereof 0.1-0.3%
Colouring agents 0-1%
Coating agents 0-0.5%.

13. The chewable composition for use in the treatment and/or prevention of postoperative paralytic ileus according to claim 10 wherein said composition is a chewing gum comprising as percentage by weight of said composition:
Gum base 25-35%
Sweeteners 53-63%
Thickeners 1-2%
Aromas 1-3%
Anti-caking agents 2.5-4.5%
Plant extracts 4.5-6.5%
Hyaluronic acid and/or a salt thereof 0.1-0.2%
Colouring agents 0-1%
Coating agents 0-0.5%.

14. The chewable composition for use in the treatment and/or prevention of postoperative paralytic ileus according to claim 10 wherein said composition is a chewable tablet comprising as percentage by weight of said composition:
Thickeners 50-70%
Sweeteners 25-35%
Anti-caking agents 4-9%
Hyaluronic acid and/or a salt thereof 0.4-0.7%
Aromas 2-3%
Colouring agents 0-0.5%.

15. The chewable composition for use in the treatment and/or prevention of postoperative paralytic ileus according to claim 10 wherein said composition is a chewable tablet comprising as percentage by weight of said composition:
Thickeners 55-70%
Sweeteners 25-35%
Anti-caking agents 4-9%
Hyaluronic acid and/or a salt thereof 0.4-0.7%
Plant extracts 4.5-6.5%
Aromas 2-3%
Colouring agents 0-0.5%.

## Patentansprüche

1. Kaubare feste oder halbfeste Zusammensetzung, die umfasst: Hyaluronsäure und/oder ein Salz davon zur Verwendung bei der Behandlung und/oder Prävention von postoperativem paralytischem Darmverschluss.

2. Kaubare Zusammensetzung zur Verwendung bei der Behandlung und/oder Prävention von postoperativem paralytischem Darmverschluss gemäß Anspruch 1, wobei die Hyaluronsäure und/oder ein Salz davon bei einer Konzentration von 0,01 bis 0,7 Gewichtsprozent der Zusammensetzung vorliegen.

3. Kaubare Zusammensetzung zur Verwendung bei der Behandlung und/oder Prävention von postoperativem paralytischem Darmverschluss gemäß Anspruch 2, wobei die Hyaluronsäure und/oder ein Salz davon bei einer Konzentration von 0,05 bis 0,5 Gewichtsprozent der Zusammensetzung, oder von 0,1 bis 0,3 Gewichtsprozent der Zusammensetzung vorliegen.

4. Kaubare Zusammensetzung zur Verwendung bei der Behandlung und/oder Prävention von postoperativem paralytischem Darmverschluss gemäß einem der Ansprüche 1 bis 3, wobei das Salz der Hyaluronsäure Natriumhyaluronat ist.

5. Kaubare Zusammensetzung zur Verwendung bei der Behandlung und/oder Prävention von postoperativem paralytischem Darmverschluss gemäß einem der Ansprüche 1 bis 4, wobei die Menge der Hyaluronsäure und/oder des Salzes davon 0,17 mg bis 12 mg pro Einzeldosis beträgt.

6. Kaubare Zusammensetzung zur Verwendung bei der Behandlung und/oder Prävention von postoperativem paralytischem Darmverschluss gemäß Anspruch 5, wobei die Menge der Hyaluronsäure und/oder des Salzes davon 0,5 bis 5,5 mg pro Einzeldosis beträgt.

7. Kaubare Zusammensetzung zur Verwendung bei der Behandlung und/oder Prävention von postoperativem paralytischem Darmverschluss gemäß einem der Ansprüche 1 bis 6, die weiterhin umfasst: einen Trockenextrakt von *Plantago Psyllium* bei einer Konzentration von 2 bis 10 Gewichtsprozent der Zusammensetzung.

8. Kaubare Zusammensetzung zur Verwendung bei der Behandlung und/oder Prävention von postoperativem paralytischem Darmverschluss gemäß Anspruch 7, wobei der Trockenextrakt bei einer Konzentration von ungefähr 4, 5, 5,5, 6, 6,5 Gewichtsprozent der Zusammensetzung vorliegt.

9. Kaubare Zusammensetzung zur Verwendung bei der Behandlung und/oder Prävention von postoperativem paralytischem Darmverschluss gemäß Anspruch 7 oder 8, wobei die Menge des Trockenextrakts 65 bis 111 mg pro Einzeldosis beträgt.

10. Kaubare Zusammensetzung zur Verwendung bei der Behandlung und/oder Prävention von postoperativem paralytischem Darmverschluss gemäß einem der Ansprüche 1 bis 9 in der Form eines Kaugummis, einer Kautablette, eines Bonbons oder einer Kapsel zur Anwendung in der Mundhöhle.

11. Kaubare Zusammensetzung zur Verwendung bei der Behandlung und/oder Prävention von postoperativem paralytischem Darmverschluss gemäß Anspruch 10, wobei die Zusammensetzung ein Kaugummi ist, das folgende Bestandteile in Gewichtsprozent der Zusammensetzung umfasst:
Gumbase 25 - 35 %
Süßstoffe 53 - 63 %
Verdickungsmittel 1 - 2 %
Aromen 1 - 3 %
Antiklumpmittel 2,5 - 4,5 %
Hyaluronsäure und/oder ein Salz davon 0,1 - 0,2 %
Farbstoffe 0 - 1 %
Überzugmittel 0 - 0,5 %.

12. Kaubare Zusammensetzung zur Verwendung bei der Behandlung und/oder Prävention von postoperativem paralytischem Darmverschluss gemäß Anspruch 10, wobei die Zusammensetzung ein Kaugummi ist, das folgende Bestandteile in Gewichtsprozent der Zusammensetzung umfasst:
Gumbase 25 - 35 %
Süßstoffe 53 - 63 %
Verdickungsmittel 1 - 2 %
Aromen 1 - 3 %
Antiklumpmittel 2,5 - 4,5 %
Hyaluronsäure und/oder ein Salz davon 0,1 - 0,3 %
Farbstoffe 0 - 1 %
Überzugmittel 0 - 0,5 %.

13. Kaubare Zusammensetzung zur Verwendung bei der Behandlung und/oder Prävention von postoperativem paralytischem Darmverschluss gemäß Anspruch 10, wobei die Zusammensetzung ein Kaugummi ist, das folgende Bestandteile in Gewichtsprozent der Zusammensetzung umfasst:
Gumbase 25 - 35 %
Süßstoffe 53 - 63 %
Verdickungsmittel 1 - 2 %
Aromen 1 - 3 %
Antiklumpmittel 2,5 - 4,5 %
Pflanzenextrakte 4,5 - 6,5 %
Hyaluronsäure und/oder ein Salz davon 0,1 - 0,2 %
Farbstoffe 0 - 1 %
Überzugmittel 0 - 0,5 %.

14. Kaubare Zusammensetzung zur Verwendung bei der Behandlung und/oder Prävention von postoperativem paralytischem Darmverschluss gemäß Anspruch 10, wobei die Zusammensetzung eine Kautablette ist, die folgende Bestandteile in Gewichtsprozent der Zusammensetzung umfasst:
Verdickungsmittel 50 - 70 %
Süßstoffe 25 - 35 %
Antiklumpmittel 4 - 9 %
Hyaluronsäure und/oder ein Salz davon 0,4 - 0,7 %
Aromen 2 - 3 %
Farbstoffe 0 - 0,5 %.

15. Kaubare Zusammensetzung zur Verwendung bei der Behandlung und/oder Prävention von postoperativem paralytischem Darmverschluss gemäß Anspruch 10, wobei die Zusammensetzung eine Kautablette ist, die folgende Bestandteile in Gewichtsprozent der Zusammensetzung umfasst:
Verdickungsmittel 55 - 70 %
Süßstoffe 25 - 35 %
Antiklumpmittel 4 - 9 %
Hyaluronsäure und/oder ein Salz davon 0,4 - 0,7 %
Pflanzenextrakte 4,5 - 6,5 %
Aromen 2 - 3 %
Farbstoffe 0 - 0,5 %.

## Revendications

1. Composition à mâcher solide ou semi-solide comprenant de l'acide hyaluronique et/ou un sel de celui-ci, pour une utilisation dans le traitement et/ou la prévention d'un iléus paralytique postopératoire.

2. Composition à mâcher pour une utilisation dans le traitement et/ou la prévention d'un iléus paralytique postopératoire selon la revendication 1, dans laquelle ledit acide hyaluronique et/ou un sel de celui-ci sont présents à une concentration comprise entre 0,01 % et 0,7 % en poids de ladite composition.

3. Composition à mâcher pour une utilisation dans le traitement et/ou la prévention d'un iléus paralytique postopératoire selon la revendication 2, dans laquelle ledit acide hyaluronique et/ou un sel de celui-ci sont présents à une concentration comprise entre 0,05 et 0,5 % en poids de ladite composition, ou entre 0,1 % et 0,3 % en poids de ladite composition.

4. Composition à mâcher pour une utilisation dans le traitement et/ou la prévention d'un iléus paralytique postopératoire selon l'une quelconque des revendications 1 à 3, dans laquelle ledit sel d'acide hyaluronique est le hyaluronate de sodium.

5. Composition à mâcher pour une utilisation dans le traitement et/ou la prévention d'un iléus paralytique postopératoire selon l'une quelconque des revendications 1 à 4, dans laquelle la quantité dudit acide hyaluronique et/ou de son sel est comprise entre 0,17 mg et 12 mg par dose unitaire.

6. Composition à mâcher pour une utilisation dans le traitement et/ou la prévention d'un iléus paralytique postopératoire selon la revendication 5, dans laquelle la quantité dudit acide hyaluronique et/ou de son sel est comprise entre 0,5 et 5,5 mg par dose unitaire.

7. Composition à mâcher pour une utilisation dans le traitement et/ou la prévention d'un iléus paralytique postopératoire selon l'une quelconque des revendications 1 à 6, comprenant en outre un extrait sec de *Plantago Psyllium* à une concentration comprise entre 2 et 10 % en poids de ladite composition.

8. Composition à mâcher pour une utilisation dans le traitement et/ou la prévention d'un iléus paralytique postopératoire selon la revendication 7, dans laquelle ledit extrait sec est à une concentration d'environ 4 %, 5 %, 5,5 %, 6 %, 6,5 % en poids de ladite composition.

9. Composition à mâcher pour une utilisation dans le traitement et/ou la prévention d'un iléus paralytique postopératoire selon la revendication 7 ou 8, dans laquelle la quantité dudit extrait sec est comprise entre 65 et 111 mg par dose unitaire.

10. Composition à mâcher pour une utilisation dans le traitement et/ou la prévention d'un iléus paralytique postopératoire selon l'une quelconque des revendications 1 à 9, sous la forme d'une gomme à mâcher, d'une tablette à mâcher, d'un bonbon ou d'une capsule à usage oro-muqueux.

11. Composition à mâcher pour une utilisation dans le traitement et/ou la prévention d'un iléus paralytique postopératoire selon la revendication 10, laquelle composition est une gomme à mâcher comprenant, en pourcentage en poids de ladite composition :
base de gomme 25 à 35 %
édulcorants 53 à 63 %
épaississants 1 à 2 %
arômes 1 à 3 %
agents anti-agglomération 2,5 à 4,5 %
acide hyaluronique et/ou un sel de celui-ci 0,1 à 0,2 %
agents colorants 0 à 1 %
agents d'enrobage 0 à 0,5 %.

12. Composition à mâcher pour une utilisation dans le traitement et/ou la prévention d'un iléus paralytique postopératoire selon la revendication 10, laquelle composition est une gomme à mâcher comprenant, en pourcentage en poids de ladite composition :
base de gomme 25 à 35 %
édulcorants 53 à 63 %
épaississants 1 à 2 %
arômes 1 à 3 %
agents anti-agglomération 2,5 à 4,5 %
acide hyaluronique et/ou un sel de celui-ci 0,1 à 0,3 %
agents colorants 0 à 1 %
agents d'enrobage 0 à 0,5 %.

13. Composition à mâcher pour une utilisation dans le traitement et/ou la prévention d'un iléus paralytique postopératoire selon la revendication 10, laquelle composition est une gomme à mâcher comprenant, en pourcentage en poids de ladite composition :
base de gomme 25 à 35 %
édulcorants 53 à 63 %
épaississants 1 à 2 %
arômes 1 à 3 %
agents anti-agglomération 2,5 à 4,5 %
extraits végétaux 4,5 à 6,5 %
acide hyaluronique et/ou un sel de celui-ci 0,1 à 0,2 %
agents colorants 0 à 1 %
agents d'enrobage 0 à 0,5 %.

14. Composition à mâcher pour une utilisation dans le traitement et/ou la prévention d'un iléus paralytique postopératoire selon la revendication 10, laquelle composition est une tablette à mâcher comprenant, en pourcentage en poids de ladite composition :
épaississants 50 à 70 %
édulcorants 25 à 35 %
agents anti-agglomération 4 à 9 %
acide hyaluronique et/ou un sel de celui-ci 0,4 à 0,7 %
arômes 2 à 3 %
agents colorants 0 à 0,5 %.

15. Composition à mâcher pour une utilisation dans le traitement et/ou la prévention d'un iléus paralytique postopératoire selon la revendication 10, laquelle composition est une tablette à mâcher comprenant, en pourcentage en poids de ladite composition :
épaississants 55 à 70 %
édulcorants 25 à 35 %
agents anti-agglomération 4 à 9 %
acide hyaluronique et/ou un sel de celui-ci 0,4 à 0,7 %
extraits végétaux 4,5 à 6,5 %
arômes 2 à 3 %
agents colorants 0 à 0,5 %.
